# EUROPÄISCHE PATENTSCHRIFT

(11) **EP 2 825 496 B1**
(45) Veröffentlichungstag und Bekanntmachung des Hinweises auf die Patenterteilung: **01.06.2016**
(21) Anmeldenummer: 13705966.3
(22) Anmeldetag: 14.02.2013
(51) Int. Cl.: B66D 1/28, E21B 19/00, B66D 1/14, B66D 1/82

(54) **HEBEWERK**
DRAWWORKS
TREUIL DE MANOEUVRE

(30) Priorität: 12.03.2012 DE 102012102046
(43) Veröffentlichungstag der Anmeldung: 21.01.2015
(73) Patentinhaber: MHWirth GmbH, 41812 Erkelenz (DE)
(72) Erfinder: HEINRICHS, Albrecht, 41812 Erkelenz (DE)
(74) Vertreter: Kluin, Jörg-Eden
(86) Internationale Anmeldenummer: PCT/EP2013/052956
(87) Internationale Veröffentlichungsnummer: WO 2013/135452

(56) Entgegenhaltungen:
- WO-A1-03/072904
- WO-A1-2005/035427
- FR-A1- 2 537 964

## Beschreibung

Die Erfindung bezieht sich auf ein Hebewerk für eine Bohreinrichtung zum Ablassen und Aufholen einer Last, insbesondere eines Bohrstranges oder Teilen desselben, mit einem Rahmen, mit einer Wickeltrommel, und mit einer mit dem Rahmen verbundenen Lagereinrichtung für die Wickeltrommel.

Bei derartigen Hebewerken ist die Last regelmäßig an einem flexiblen Zugelement, welches meist als Stahlseil ausgebildet ist, befestigt. Zum Ablassen und Aufholen wird die Wickeltrommel derart drehangetrieben, dass das flexible Zugmittel ab- oder aufgewickelt wird.

Bei Hebewerken gemäß dem Stand der Technik, wie sie beispielsweise aus der WO 03/072904 A1 bekannt sind, ist die Wickeltrommel drehfest auf einer Wickelwelle befestigt, deren Achse etwa parallel zu den Längsseiten des Rahmens verläuft. Sie ist in Lagerböcken gelagert, die jenseits der beiden Stirnseiten der Wickeltrommel fest mit dem Rahmen verbunden sind. Von der Wickeltrommel aus gesehen jenseits des einen Lagerbocks ist eine auf die Wickelwelle wirkende Bremseinrichtung vorgesehen. Sie ist regelmäßig als Scheibenbremseinrichtung ausgebildet und umfasst eine mit der Wickelwelle drehfest verbundene Bremsscheibe sowie an dem Rahmen befestigte Bremszangenanordnungen, sogenannte "Kalipper". Die Bremsscheibe kann auch direkt an der Trommelbordscheibe angeordnet sein.

Die FR 2 537 964 A1 zeigt eine Seilwinde, bei der die Welle der Wickeltrommel auf der einen Seite mittels genau einem Loslager und auf der anderen Seite mittels zweier Lager gelagert ist, die zwar schwenkbar sind, jedoch keine translatorische Bewegung in Richtung der Welle zulassen.

Die WO 2005/035427 A1 offenbart ein Hebewerk, bei welchem eine Bremsanordnung auf der der Getriebeeinheit gegenüberliegenden Seite der Wickeltrommel angeordnet ist.

Von der Wickeltrommel aus gesehen jenseits des anderen Lagerbocks ist eine Getriebeeinheit vorgesehen, die bei dem aus der WO 03/072904 A1 bekannten Ausführungsbeispiel zwei einzelne Schaltgetriebe sowie ein mit der Wickelwelle verbundenes Summengetriebe umfasst. Die Schaltgetriebe sind fest mit dem Rahmen verbunden. Das Gehäuse des Summengetriebes ist über eine Drehmomentstütze mit dem Rahmen verbunden.

Um zu vermeiden, dass es bei Verwindungen des Rahmens, die nicht zu vermeiden sind, wenn sich beispielsweise der Untergrund, auf dem der Rahmen gelagert ist, setzt, oder die bei Temperaturänderungen auftreten können, zu den Verschleiß erhöhenden oder sogar das gesamte Hebewerk beschädigenden Verspannungen in der Wickelwelle und der Getriebeanordnung kommt, sind die Schaltgetriebe über elastische Kupplungen mit dem Summengetriebe verbunden.

Bei einem anderen, von der Anmelderin bekannten Hebewerk ist das Summengetriebe ebenfalls mit dem Rahmen fest verbunden. Eine elastische Kupplung ist dann zwischen der Abtriebswelle des Summengetriebes und der Wickelwelle vorgesehen.

Obwohl aufgrund der elastischen Kupplungen gewisse Verwindungen des Rahmens ausgeglichen werden können, ist bei diesen Hebewerken darauf zu achten, dass der Rahmen auf dem Untergrund im Sinne eines Fluchtens bzw. Parallellaufens sämtlicher Achsen und Wellen des Hebewerks genau ausgerichtet ist, da die Kupplungen lediglich begrenzte Abweichungen aus den parallelen bzw. fluchtenden Ausrichtungen der Achsen bzw. Wellen zulassen und die Belastungen der Kupplungen mit Zunahme von Abweichungen steigt. Daher muss der Rahmen einerseits möglichst verwindungssteif ausgebildet sein, was sowohl sein Gewicht, als auch seine Fertigungskosten unvorteilhafterweise erhöht. Darüber hinaus hat die Erfahrung gezeigt, dass der Rahmen im Laufe des Betriebs eines Hebewerks regelmäßig neu ausgerichtet werden muss, da sich der Untergrund aufgrund der dynamischen Lastwechsel, die beim Heben und Senken von Lasten auf das Hebewerk wirken, setzt.

Der Erfindung liegt daher die Aufgabe zugrunde, ein hinsichtlich dieser Problematik verbessertes Hebewerk zu schaffen.

Diese Aufgabe wird durch das in Anspruch 1 wiedergegebene Hebewerk gelöst. Bei dem erfindungsgemäßen Hebewerk ist eine Lagereinrichtung für die Wickeltrommel vorgesehen, die an genau drei Lagerstellen mit dem Rahmen verbunden ist. Aufgrund dieser "Drei-Punktlagerung" haben eventuelle Verwindungen des Rahmens nur noch geringere Auswirkungen auf die Justierung der Wickelwelle.

Es ist genau eine der drei Lagerstellen auf derjenigen Seite der Wickeltrommel vorgesehen, auf welcher eine Bremseinrichtung für die Wickeltrommel vorgesehen ist. Ist die Wickeltrommel - wie in den meisten Fällen - drehfest auf einer Wickelwelle befestigt, so kann die Lagerstelle derart angeordnet und ausgebildet sein, dass sie die Wickelwelle in einem geeigneten Drehlager aufnimmt. In einem Bereich der Wickelwelle, die auf der von der Wickeltrommel fort weisenden Seite aus dem Drehlager herausragt, kann ein Teil einer Bremseinrichtung, beispielsweise eine Bremsscheibe einer Scheibenbremseinrichtung vorgesehen sein. Die Lageranordnung für die Wickelwelle ist so ausgebildet, dass die Wickelwelle in axialer Richtung der Wickelwelle fest, jedoch bevorzugt um senkrecht zur Wickelwelle verlaufende Achsen verschwenkbar gelagert ist. Durch die in axialer Richtung feste Lagerung wird sichergestellt, dass zwischen den Bremsbacken und der Bremsscheibe kein eine Bremsscheibenbewegung in axialer Richtung erlaubender Spalt vorhanden sein muss, durch welchen der Betätigungsweg der Bremsbacken unvorteilhaft verlängert würde, sofern nicht die Bremszangen ebenfalls in einer zur Längsachse der Wickelwelle parallelen Richtung verlagerbar, mit anderen Worten "schwimmend" gelagert wären.

Die erste Lageranordnung kann, um die Verschwenkbarkeit um senkrecht zur Wickelwelle verlaufende Achsen zu bewirken, ein Pendellager, vorzugsweise ein Pendelrollenlager umfassen.

Die beiden anderen Lagerstellen der Lagereinrichtung sind auf der Seite der Wickeltrommel vorgesehen, auf welcher eine Drehantriebseinrichtung für die Wickeltrommel angeordnet ist. Diese beiden Lagerstellen sind vorzugsweise auf einander gegenüberliegenden Seiten der Längsachse der Wickelwelle, d.h. bei einer Ansicht senkrecht auf den Rahmen beidseitig der Wickeltrommel angeordnet.

Die beiden Lagerstellen können von einer zweiten Lagervorrichtung gebildet sein, in der der drehantriebsseitige Bereich der Wickelwelle gelagert ist. Die Lagervorrichtung ist vorzugsweise derart ausgebildet, dass in der zur Wickelwelle parallelen Richtung eine Verlagerung der Wickelwelle relativ zum Rahmen sowie um senkrecht zur Wickelwelle verlaufende Achsen Verschwenkungen möglich sind. Die zweite Lagervorrichtung ist vorzugsweise an einer Antriebseinheit vorgesehen, die insbesondere eine Drehantriebsleistung auf die Wickelwelle übertragende Getriebeeinrichtung sowie mindestens einen mit der Getriebeeinrichtung wirkverbundenen Rotationsmotor umfasst. Aufgrund dieser Maßnahme, die erst durch die erfindungsgemäße "Drei-Punktlagerung" ermöglicht wird, kann auf Verwindungen des Rahmens in einem gewissen Umfange zulassende, elastische Kupplungen verzichtet werden. Die Drehantriebseinheit kann direkt, d.h. ohne Zwischenschaltung einer oder mehrer elastischer Kupplungen, mit der Wickelwelle verbunden sein. Eventuelle Verwindungen des Rahmens oder Verschiebungen in Richtung der Längsachse der Wickelwelle führen gleichwohl nicht zu Spannungen in der Wickelwelle, da die Drehantriebseinrichtung nicht mehr durch Verwindungen oder thermische Ausdrehungen des Rahmens relativ zur Wickelwelle verlagert wird.

Jede der zwei auf der Seite der Drehantriebseinrichtung für die Wickeltrommel vorgesehenen Lagerstellen umfasst eine Längsverschiebungen parallel zur axialen Richtung der Wickelwelle und bevorzugt Verschwenkungen um senkrecht zur Wickelwelle verlaufende Achsen erlaubende zweite Lageranordnung.

Jede zweite Lageranordnung kann ein Pendellager, vorzugsweise ein Pendelgleitlager aufweisen.

Um die Verschiebung in einer zur Längsachse der Wickelwelle parallelen Richtung zu erlauben, kann jede zweite Lageranordnung einen mit der Antriebseinrichtung verbundenen Lagerbolzen umfassen, der von dem Pendelgleitlager in zur axialen Richtung der Wickelwelle parallelen Richtung verschiebbar aufgenommen ist.

In der Zeichnung ist ein Ausführungsbeispiel des erfindungsgemäßen Hebewerks - schematisch - dargestellt.

Es zeigen:

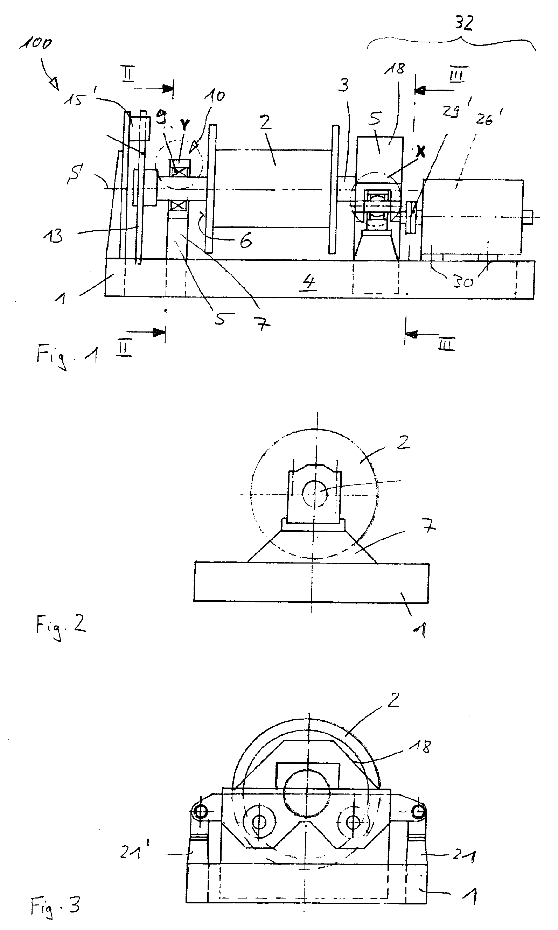

- Fig. 1: eine seitliche Ansicht dieses Hebewerks senkrecht zur Mittellängsachse der Wickelwelle;
- Fig. 2: die Ansicht II-II in Fig. 1;
- Fig. 3: die Ansicht III-III in Fig. 1;
- Fig. 4: eine Ansicht gemäß Fig. 1 von oben;
- Fig. 5: die Position der Lagerkraftzentren der bei dem erfindungsgemäßen Hebewerk verwirklichten "Drei-Punktauflage" auf dem Rahmen;
- Fig. 6: die Einzelheit Y in Fig. 1 in einer vergrößerten Darstellung sowie
- Fig. 7: die Einzelheit X in Fig. 1 in einer vergrößerten Darstellung, teilgeschnitten.

Das in der Zeichnung als Ganzes mit 100 bezeichnete Ausführungsbeispiel eines erfindungsgemäßen Hebewerks umfasst einen Rahmen 1, auf dem die wesentlichen Bauteile des Hebewerks 100 montiert sind. Zum Aufholen und Ablassen sowie je nach Einsatzbedingungen auch zum Halten einer Last, insbesondere eines Bohrgeräts, eines Bohrstranges oder von Teilen desselben umfasst das Hebewerk 100 eine Wickeltrommel 2, die drehfest auf einer Wickelwelle 3 befestigt ist und deren Mittellängsachse S parallel zu den Längsseiten 4 des Rahmens 1 verläuft.

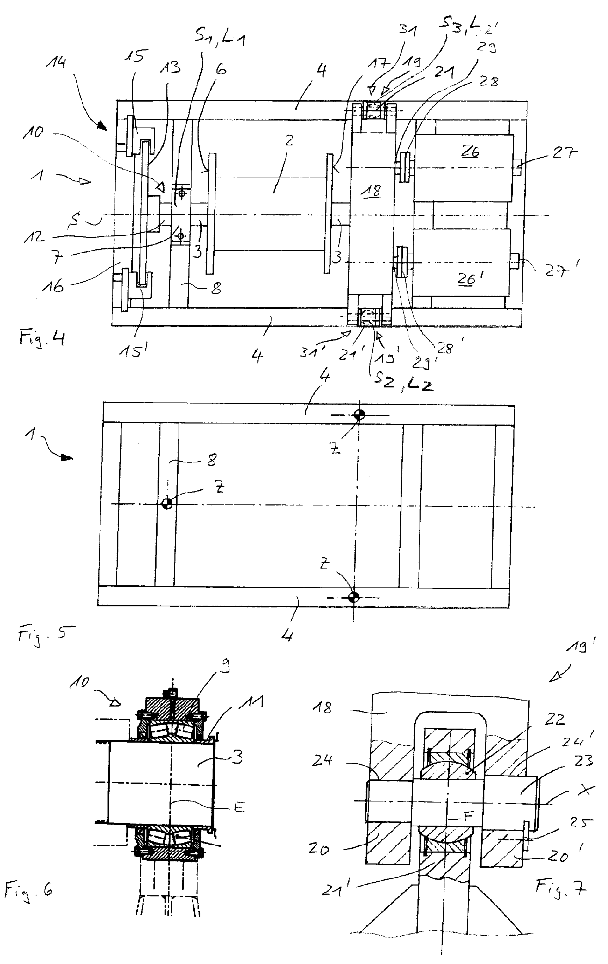

Zur Lagerung der Wickelwelle 3 umfasst das Hebewerk 100 eine als Ganzes mit 5 bezeichnete Lagereinrichtung, die als "Drei-Punktlagerung" ausgebildet ist, so dass sich die Zentren Z (s. Fig. 5) der Lagerkräfte an drei Stellen S₁, S₂, S₃ des Rahmens 1 befinden.

Hierzu weist die Lagereinrichtung 5 bei dem in der Zeichnung dargestellten Ausführungsbeispiel links von der linken Stirnseite 6 der Wickeltrommel einen Lagerbock 7 auf, welcher mit einer Quertraverse 8 des Rahmens 1 fest verbunden ist (s. insbesondere Fig. 4). Der Lagerbock 7 umfasst eine als Pendelrollenlager 9 ausgebildete erste Lageranordnung 10. Diese umfasst, wie in Fig. 6 erkennbar ist, eine innere Lagerschale 11, die fest mit der Wickelwelle 3 verbunden ist. Die Wickelwelle 3 wird somit in Richtung der Achse S mit Hilfe des Pendelrollenlagers fixiert, ist jedoch um Achsen senkrecht zur Achse S, die in der Lagerebene E liegen, verschwenkbar. Die Wickelwelle 3 durchsetzt das Pendelrollenlager 9. An dem von der Wickeltrommel 2 fort weisenden Bereich 12 ist eine Scheibenbremse 13 einer Scheibenbremsanordnung 14 befestigt. Letztere umfasst darüber hinaus zwei Bremszangen 15, 15' oder mehr, die fest mit einer weiteren Quertraverse 16 des Rahmens 1 verbunden sind.

Jenseits der in Fig. 4 rechts dargestellten Stirnseite 17 der Wickeltrommel 2 ist die Wickelwelle 3 in einer Lagervorrichtung 18 rotierbar gelagert, die einen weiteren Teil der Lagereinrichtung 5 bildet. Wie insbesondere in Fig. 4 erkennbar ist, umfasst die Lagereinrichtung 18 zwei beidseitig der Mittellängsachse S der Wickelwelle 3 angeordnete gabelförmige Aufnahmen 19, 19'. Wie in Fig. 7 erkennbar ist, welche die gabelförmige Aufnahme 19' in einer vergrößerten Einzelteildarstellung zeigt, greift zwischen die beiden Zinken 20, 20' der Aufnahme ein Lagerbock 21, 21' ein. Beide Lagerböcke 21, 21' sind mit jeweils einer Rahmenseite 4 fest verbunden. Jeder Lagerbock 21, 21' nimmt ein als Gleitlager ausgebildetes Pendellager 22 auf. Zur Verbindung der Lagervorrichtung 18 mit den Lagerböcken 21, 21' ist jeweils ein das jeweilige Pendellager 22 durchsetzender Stufenbolzen 23 vorgesehen, der bei dem in der Zeichnung dargestellten Ausführungsbeispiel (s. insbesondere Fig. 7) von rechts in hierfür vorgesehene Augen 24, 24' und durch das Pendellager 22 hindurch eingeschoben und mit Hilfe eines Arretierelements 25 in seiner Längsrichtung festgelegt ist. Der Stufenbolzen 23 durchsetzt das Pendellager mit seinem mittleren Bereich gleitend. Da der Abstand zwischen den beiden Zinken 20, 20' größer als die Breite des jeweiligen Lagerbocks 21, 21' ist, kann in der Größe der Differenz zwischen Abstand und Breite eine Verschiebung zwischen der Lagervorrichtung 18 und den Lagerböcken 21, 21' in Richtung der Stufenbolzenachse X erfolgen. Ferner können Verschwenkungen um senkrecht zur Stufenbolzenachse X und in der Lagerebene F liegende Achsen zwischen dem Stufenbolzen 23 und damit der Lagervorrichtung 18 erfolgen. Insofern ist bei dem erfindungsgemäßen Hebewerk eine Verwindung des Rahmens 1 in einem Umfange möglich, der durch die Konzipierung der Pendellager 22 und des Pendelrollenlagers 9 begrenzt ist, der nicht zu Verspannungen der Wickelwelle führt.

Bei dem in der Zeichnung dargestellten Ausführungsbeispiel eines erfindungsgemäßen Hebewerks 100 ist die Lagervorrichtung 18 als Summengetriebe ausgebildet. Die Wickelwelle 3 ist die Getriebeabtriebswelle oder ist mit dieser drehfest verbunden und wird von ihr gelagert. Eine elastische Kupplung zur Verbindung der Getriebeabtriebswelle und der Wickelwelle 3 sind daher nicht nötig. Der mit der Herstellung und dem Einbau der elastischen Kupplung verbundene Aufwand muss daher bei dem erfindungsgemäßen Hebewerk nicht betrieben werden. Ferner verkürzt sich durch den Entfall der elastischen Kupplung in vorteilhafter Weise die Baulänge des erfindungsgemäßen Hebewerks.

Bei dem in der Zeichnung dargestellten Ausführungsbeispiel des erfindungsgemäßen Hebewerks sind auf der der Wickeltrommel 2 gegenüberliegenden Seite der das Getriebe umfassenden Lagervorrichtung 18 zwei Drehantriebsmotoren 26, 26' vorgesehen. Sie können insbesondere elektrisch, pneumatisch oder hydraulisch antreibbar ausgebildet sein. Im gezeigten Ausführungsbeispiel handelt es sich um Elektromotoren. Um eine elektrische Isolierung zwischen den Antriebswellen 27, 27' vom Rest des Hebewerks 100 sicherzustellen, sind die Antriebswellen über isolierende Kupplungen 28, 28' mit den Eingangswellen 29, 29' der als Summengetriebe ausgebildeten Lagervorrichtung 18 verbunden. Insbesondere, wenn anstatt von Elektromotoren hydraulisch oder pneumatisch betriebene Drehantriebsmotoren 26, 26' Verwendung finden, kann auf die isolierenden Kupplungen verzichtet werden. Da derart betriebene Motoren regelmäßig auch eine kleinere Bauform und ein geringeres Gewicht bei mit Elektromotoren vergleichbarer Leistung aufweisen, können diese dann auch direkt an die Lagervorrichtung 18 angebracht werden. Es bedarf dann keiner weiteren Befestigung 30 an dem Rahmen, wie dies bei den in dem dargestellten Ausführungsbeispiel vorhandenen Elektromotoren der Fall ist.

Der Vollständigkeit halber sei erwähnt, dass anstelle von zwei Antriebsmotoren auch lediglich einer oder auch mehr als zwei vorgesehen sein können. Auch kann der oder können die Antriebsmotoren auf einem von dem Rahmen 1 separaten Rahmen angeordnet und über längere Wellen mit der das Getriebe umfassenden Lagervorrichtung 18 verbunden sein. Das Getriebe kann ein oder mehrstufig und als Eingang- oder Mehrgangausführung gebaut sein. Das Getriebe kann in die Lagervorrichtung 18 integriert oder auch an diese angeflanscht sein. Auch ist es denkbar, langsam laufende Drehantriebe an der Lagervorrichtung 18 zu befestigen und direkt mit der Wickelwelle 3 zu verbinden.

Auch müssen die an der Lagervorrichtung 18 vorgesehenen zweiten Lageranordnungen 31, 31' nicht, wie in dem in der Zeichnung dargestellten Ausführungsbeispiel, symmetrisch zur Mittellängsachse S der Wickelwelle 3 angeordnet sein.

Zusammenfassend weist ein erfindungsgemäßes Hebewerk die folgenden Vorteile auf:
- Aufgrund der realisierten Drei-Punktlagerung werden Verspannungen in der Wickelwelle und im Getriebe vermieden.
- Eine Ausrichtung des Rahmens muss nicht mehr so genau wie bei zum Stand der Technik gehörenden Hebewerken erfolgen, so dass der Aufbau und die Installation des erfindungsgemäßen Hebewerks vereinfacht ist.
- Der Rahmen des Hebewerks kann weniger stabil und damit leichter ausgebildet sein.
- Aufgrund einer geringeren Anzahl von Bauteilen sind der Zusammenbau und auch das Zerlegen eines erfindungsgemäßen Hebewerks vereinfacht.
- Wegen des Verzichts auf ansonsten teilweise erforderliche elastische Kupplungen kann die Baulänge des erfindungsgemäßen Hebewerks verkürzt werden.

### Bezugszeichenliste:

- 100: Hebewerk
- 1: Rahmen
- 2: Wickeltrommel
- 3: Wickelwelle
- 4: Rahmenseite
- 5: Lagereinrichtung (Getriebeeinheit)
- 6: Linke Stirnseite
- 7: Lagerbock
- 8: Quertraverse
- 9: Pendelrollenlager
- 10: erste Lageranordnung
- 11: innere Lagerschale
- 12: Bereich
- 13: Scheibenbremse
- 14: Scheibenbremsanordnung
- 15, 15': Bremszangen
- 16: Quertraverse
- 17: rechte Stirnseite
- 18: Lagervorrichtung (Getriebeeinheit)
- 19, 19': Aufnahmen
- 20, 20': Zinken
- 21, 21': Lagerböcke
- 22: Pendellager
- 23: Stufenbolzen
- 24, 24': Augen
- 25: Arretierelement
- 26, 26': Drehantriebsmotoren
- 27, 27': Antriebswellen
- 28, 28': Kupplungen
- 29, 29': Eingangswellen
- 30: Befestigung
- 31, 31': zweite Lageranordnungen
- 32: Drehantriebseinrichtung

- E: Lagerebene
- F: Lagerebene
- S: Achse
- S₁, S₂, S₃: Lagerstellen
- X: Stufenbolzenachse
- L₁: erste Lagerstelle
- L₂, L_{2'}: zweite Lagerstellen
- Z: Zentren

## Patentansprüche

1. Hebewerk (100) für eine Bohreinrichtung zum Ablassen und Aufholen einer Last, insbesondere eines Bohrstranges oder Teilen desselben,
mit einem Rahmen (1),
mit einer Wickeltrommel (2),
und mit einer mit dem Rahmen (1) verbundenen Lagereinrichtung (5) für die Wickeltrommel (2),
wobei
die Lagereinrichtung (5) derart ausgebildet ist, dass sich die Zentren (Z) der Lagerkräfte an genau drei Lagerstellen (S₁, S₂, S₃) des Rahmens (1) befinden, **dadurch gekennzeichnet, dass** genau eine erste Lagerstelle (S₁) auf der Seite des Hebewerks (100) vorgesehen ist, auf welcher eine Bremseinrichtung für die Wickeltrommel (2) angeordnet ist
und genau zwei Lagerstellen (L₂, L_{2'}) auf der Seite des Hebewerks vorgesehen sind, auf welcher eine Drehantriebseinrichtung (32) für die Wickeltrommel (2) angeordnet ist,
wobei die auf der Seite der Bremseinrichtung vorgesehene Lagerstelle (L₁) eine erste Lageranordnung (10) umfasst, in der eine Wickelwelle (3), mit der die Wickeltrommel (2) drehfest verbunden ist, in axialer Richtung der Wickelwelle (3) fest gelagert ist,
wobei jede der zwei auf der Seite der Drehantriebseinrichtung (32) für die Wickeltrommel (2) vorgesehenen Lagerstellen (L₂, L₂') eine Längsverschiebung parallel zur axialen Richtung (S) der Wickelwelle (3) erlaubende zweite Lageranordnung (31, 31') umfasst.

2. Hebewerk nach Anspruch 1, **dadurch gekennzeichnet, dass** die Wickelwelle (3), mit der die Wickeltrommel (2) drehfest verbunden ist, um senkrecht zur Wickelwelle (3) verlaufenden Achsen in der ersten Lageranordnung (10) verschwenkbar gelagert ist und die erste Lageranordnung (10) ein Pendellager, vorzugsweise ein Pendelrollenlager (9) umfasst.

3. Hebewerk nach Anspruch 1 oder 2, **dadurch gekennzeichnet, dass** die zwei auf der Seite der Drehantriebsrichtung (32) für die Wickeltrommel (2) vorgesehenen Lagerstellen (L₂, L_{2'}) von einer Lagervorrichtung (18) gebildet sind, in der der drehantriebsseitige Bereich der Wickelwelle (3) gelagert ist und die in der zur Wickelwelle (3) parallelen Richtung eine Verlagerung der Wickelwelle (3) relativ zum Rahmen (1) sowie um senkrecht zur Wickelwelle (3) verlaufenden Achsen Verschwenkungen erlaubt.

4. Hebewerk nach Anspruch 3, **dadurch gekennzeichnet, dass** die zweite Lagervorrichtung (18) an einer Antriebseinheit vorgesehen ist, die insbesondere eine Drehantriebsleistung auf die Wickelwelle (3) übertragende Getriebeeinrichtung umfasst.

5. Hebewerk nach einem der Ansprüche 1 bis 4, **dadurch gekennzeichnet, dass** jede zweite Lageranordnung (31, 31') Verschwenkungen um senkrecht zur Wickelwelle (3) verlaufenden Achsen erlaubt und ein Pendellager (22), vorzugsweise ein Pendelgleitlager umfasst.

6. Hebewerk nach einem der Ansprüche 1 bis 5, **dadurch gekennzeichnet, dass** jede zweite Lageranordnung (31, 31') einen mit der Lagervorrichtung verbundenen oder in diese eingesetzten Bolzen (23) umfasst, der von dem Pendellager in zur axialen Richtung der Wickelwelle (3) parallelen Richtung verschiebbar aufgenommen ist.

## Claims

1. Draw works (100) for a drilling attachment to lower and lift a load, in particular a drill string or parts of the same, provided
with a frame (1),
with a winding drum (2),
and with a bearing device (5) connected to the frame (1) for the winding drum (2), wherein
the bearing device (5) is designed in such a way that the centers (Z) of the bearing forces are arranged on precisely three bearing points (S₁, S₂, S₃) of the frame (1), **characterized in that** precisely a first bearing point (S₁) is provided on the side of the draw works (100) on which a braking device for the winding drum (2) is arranged
and precisely two bearing points (L₂, L_{2'}) are provided on the side of the draw works on which a rotary drive unit (32) for the winding drum (2) is arranged,
wherein the bearing point (L₁) comprises a first bearing arrangement (10), in which a winding shaft (3), to which the winding drum (2) is connected non-rotatably, is mounted fixed in axial direction of the winding shaft (3),
wherein each of the two bearing points (L₂, L_{2'}) provided for the winding drum (2) on the side of the rotary drive unit (32) comprises a second bearing arrangement (31, 31') which permits longitudinal displacement parallel to the axial direction (S) of the winding drum (3).

2. Draw works according to claim 1, **characterized in that** the winding shaft (3), to which the winding drum (2) is connected non-rotatably, is mounted rotatably on the axes 2 extending vertically to the winding shaft (3) in the first bearing arrangement (10), 2 and the first bearing arrangement (10) comprises a pendulum bearing, preferably a pendulum roller bearing (9).

3. Draw works according to claims 1 or 2, **characterized in that** the two bearing points (L₂, L_{2'}) provided for the winding drum (2) on the side of the rotatory drive unit (32) are formed by a bearing device (18), in which the area of the winding shaft (3) is mounted on the side of the rotary drive and which, in the direction parallel to the winding shaft (3), permits a displacement of the winding shaft (3) relative to the frame (1) and also pivoting around the axes extending vertically to the winding shaft (3).

4. Draw works according to claim 3, **characterized in that** the second bearing device (18) is provided on a drive unit, which comprises in particular a transmission unit transferring a rotary drive power to the winding shaft (3).

5. Draw works according to claims 1 to 4, **characterized in that** each second bearing arrangement (31, 31') permits pivoting around axes extending vertically to the winding shaft (3) and comprises a pendulum bearing (22), preferably a pendulum plain bearing.

6. Draw works according to claims 1 to 5, **characterized in that** each second bearing arrangement (31, 31') comprises a bolt (23) connected to or inserted in the bearing device, which bolt is seated movably in the pendulum bearing in the direction that is parallel to the axial direction of the winding shaft (3).

## Revendications

1. Treuil (100) pour un dispositif de forage, pour faire descendre et remonter une charge, en particulier un train de forage ou des parties de celui-ci,
avec un cadre (1),
avec un tambour d'enroulement (2),
et avec un dispositif de support (5) relié au cadre (1) pour le tambour d'enroulement (2),
dans lequel
le dispositif de support (5) est conçu de telle façon que les centres (Z) des forces de support se situent exactement en trois points de support (S₁, S₂, S₃) du cadre (1), **caractérisé en ce qu'**un premier point de support (S₁) exactement est prévu sur le côté du treuil (100) sur lequel est agencé un dispositif de freinage pour le tambour d'enroulement (2),
et deux points de support (L₂, L_{2'}) exactement sont prévus sur le côté du treuil sur lequel est agencé un dispositif d'entraînement en rotation (32) pour le tambour d'enroulement (2),
dans lequel le point de support (L₁) prévu sur le côté du dispositif de freinage comprend un premier ensemble de support (10), dans lequel un arbre d'enroulement (3) auquel le tambour d'enroulement (2) est relié solidairement en rotation est monté fixement dans la direction axiale de l'arbre d'enroulement (3),
dans lequel chacun des deux points de support (L₂, L_{2'}) prévus sur le côté du dispositif d'entraînement en rotation (32) pour le tambour d'enroulement (2) comprend un deuxième ensemble de support (31, 31') permettant un déplacement longitudinal parallèlement à la direction axiale (S) de l'arbre d'enroulement (3).

2. Treuil selon la revendication 1, **caractérisé en ce que** l'arbre d'enroulement (3) relié solidairement en rotation au tambour d'enroulement (2) est monté de façon à pouvoir pivoter dans le premier ensemble de support (10) autour d'axes s'étendant perpendiculairement à l'arbre d'enroulement (3), et le premier ensemble de support (10) comprend un palier oscillant, de préférence un palier oscillant à rouleaux (9).

3. Treuil selon la revendication 1 ou 2, **caractérisé en ce que** les deux points de support (L₂, L_{2'}) prévus sur le côté du dispositif d'entraînement en rotation (32) pour le tambour d'enroulement (2) sont formés par un dispositif de support (18) dans lequel est montée la région de l'arbre d'enroulement (3) située du côté de l'entraînement en rotation, et lequel permet un déplacement de l'arbre d'enroulement (3) par rapport au cadre (1) dans la direction parallèle à l'arbre d'enroulement (3), ainsi que des pivotements autour d'axes s'étendant perpendiculairement à l'arbre d'enroulement (3).

4. Treuil selon la revendication 3, **caractérisé en ce que** le deuxième dispositif de support (18) est prévu sur une unité d'entraînement comprenant en particulier un dispositif d'engrenage transmettant une puissance d'entraînement en rotation à l'arbre d'enroulement (3) .

5. Treuil selon l'une des revendications 1 à 4, **caractérisé en ce que** chaque deuxième ensemble de support (31, 31') permet des pivotements autour d'axes s'étendant perpendiculairement à l'arbre d'enroulement (3) et comprend un palier oscillant (22), de préférence un palier lisse oscillant.

6. Treuil selon l'une des revendications 1 à 5, **caractérisé en ce que** chaque deuxième ensemble de support (31, 31') comprend un boulon (23) relié au dispositif de support ou inséré dans celui-ci, lequel est reçu par le palier oscillant de façon à pouvoir coulisser dans une direction parallèle à la direction axiale de l'arbre d'enroulement (3).
